(19) Europäisches Patentamt
European Patent Office
Office européen des brevets

(11) EP 2 897 578 B1

(12) **EUROPEAN PATENT SPECIFICATION**

(45) Date of publication and mention of the grant of the patent:
**11.04.2018 Bulletin 2018/15**

(21) Application number: **13770822.8**

(22) Date of filing: **20.09.2013**

(51) Int Cl.:
*A61Q 15/00* (2006.01) *A61K 8/37* (2006.01)
*A61K 8/58* (2006.01) *A61Q 13/00* (2006.01)
*A61K 8/89* (2006.01) *A61K 8/02* (2006.01)
*A61K 8/11* (2006.01) *A61K 8/31* (2006.01)
*A61K 8/81* (2006.01) *A61K 8/86* (2006.01)

(86) International application number:
**PCT/US2013/061022**

(87) International publication number:
**WO 2014/047502 (27.03.2014 Gazette 2014/13)**

(54) **ANHYDROUS COMPOSITIONS HAVING MICROCAPSULES AND NON-VOLATILE OILS**

WASSERFREIE ZUSAMMENSETZUNGEN MIT MIKROKAPSELN UND NICHT-FLÜCHTIGEN ÖLEN

COMPOSITIONS ANHYDRES COMPRENANT DES MICROCAPSULES ET DES HUILES NON VOLATILES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2012 US 201261703575 P**
**20.09.2012 US 201261703616 P**

(43) Date of publication of application:
**29.07.2015 Bulletin 2015/31**

(73) Proprietor: **The Procter & Gamble Company**
**Cincinnati, OH 45202 (US)**

(72) Inventors:
- **CETTI, Jonathan, Robert**
  **Cincinnati, Ohio 45202 (US)**
- **WITT, Steven, Edward**
  **Cincinnati, Ohio 45202 (US)**
- **DIHORA, Jiten, Odhavji**
  **Cincinnati, Ohio 45202 (US)**
- **LI, Jianjun, Justin**
  **Cincinnati, Ohio 45202 (US)**

(74) Representative: **Sauvaître, Thibault Bruno**
**Procter & Gamble Service GmbH**
**IP Department**
**Sulzbacher Straße 40-50**
**65824 Schwalbach am Taunus (DE)**

(56) References cited:
**EP-A2- 2 433 617**
**EP-A2- 2 620 211**
**DE-A1-102008 035 013**
**DE-A1-102008 035 014**
**GB-A- 2 274 989**
**JP-A- 2009 280 533**
**US-A1- 2010 104 612**
**US-A1- 2012 076 839**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD

The present disclosure generally relates to anhydrous compositions having microcapsules, and specifically relates to anhydrous compositions having microcapsules and nonvolatile oils.

### BACKGROUND

Some personal care products, such as antiperspirants and deodorants, have anhydrous compositions that include non-volatile oils. These non-volatile oils can provide various benefits regarding product feel, stick hardness, application residue, etc. Some personal care products are also known to include some types of microcapsules.

A microcapsule may be a micro-sized structure. Many microcapsules have an overall size that may be measured in micrometers. A microcapsule typically has a shell that encapsulates a core material. Microcapsules can be used to encapsulate various substances. For example, a microcapsule can be used to encapsulate a fragrance.

The shell of a microcapsule may function as a diffusion barrier that surrounds the core. The shell of a microcapsule can be made from various materials. A microcapsule is useful for isolating the core material from its surroundings, until the core material is ready to be released. Depending on the kind of microcapsule, the core material can be released in various ways. One kind of microcapsule is a friable microcapsule. A friable microcapsule is configured to release the core material when its shell is ruptured. The rupture can be caused by forces applied to the shell during mechanical interactions.

U.S. Patent 2010/104612 A1 is related to an anhydrous antiperspirant composition comprising particulate antiperspirant active, water-insoluble, dry particulate friction-sensitive capsules of perfume, and a liquid carrier for the particulate antiperspirant active and capsules of perfume comprising at least one water-immiscible oil.

UK Patent Application GB 2 274 989 A refers to non-aqueous perfuming composition with deodorant or antiperspirant action, intended for body care, and containing an active deodorant or antiperspirant base and a perfuming base.

Japanese Patent Application JP 2009 280533 concerns a skin cosmetic comprising a microencapsulated cooling agent in which an oil phase comprising (A) 1-menthol and (B) a liquid oil is held in an outer-shell base which contains a water-soluble polymer compound as a major ingredient.

German Patent Applications DE 10 2008 035013 A1 and DE 10 2008 035014 A1 relate to anhydrous deodorant compositions which are applied as a non-aerosol and contain encapsulated aromatic alcohols.

European Patent Application EP 2 433 617 A2 concerns a cosmetic composition comprising (a) microcapsules, whose capsule walls include a resin obtained by reacting (i) at least one aromatic alcohol or its ether or derivative, (ii) at least one aldehyde component having at least two carbon atoms per molecule, and (iii) optionally in the presence of at least one (meth)acrylate polymers; and (b) at least one cosmetic active ingredient.

For example, a deodorant may include an anhydrous composition that includes friable fragrance microcapsules. When the anhydrous composition is applied to a user's underarm, the user's arm movements and skin may press against the composition. The pressure from such a press may be sufficient to rupture the some of the shells of the microcapsules, ultimately releasing the fragrance that was encapsulated by the shells. However, such deodorants containing microcapsules at times may not deliver a sufficient amount of fragrance throughout the period of use. Thus, there exists a need for improved processes and compositions containing such microcapsules.

### SUMMARY

An anhydrous composition, comprising: a plurality of microcapsules, wherein the microcapsules comprise a core material and a shell encapsulating the core material; and from 10% to 23%, by total mass of the anhydrous composition, of non-volatile oils; wherein the core material comprises a first fragrance; wherein the shell includes a polyacrylate material, wherein the microcapsules are spray-dried microcapsules; and wherein the anhydrous composition further comprises a moisture-triggered fragrance delivery system.

### DETAILED DESCRIPTION OF THE INVENTION

The composition can be an anhydrous composition. The term "anhydrous" as used herein means that the composition and the essential or optional components thereof, are free of added or free water. From a formulation standpoint, this means that the anhydrous compositions contains zero percent, by mass of free or added water, other than the water of hydration typically associated with the particulate antiperspirant active and/or the spray dried microcapsules prior to formulation. The anhydrous composition can be any kind of composition disclosed herein or known in the art. For example, the anhydrous composition can be a personal care composition such as a semi-solid deodorant,

semi-solid antiperspirant, an invisible solid deodorant, an invisible solid antiperspirant, aerosol antiperspirant, fluid antiperspirant, body powder, and foot powder. A parent fragrance may be a fragrance that is dispersed throughout the composition and is typically not encapsulated when added to the composition and/or article. Herein, a non-parent fragrance refers to a fragrance that differs from a parent fragrance included within the composition and/or article. Non-limiting examples of differences between a fragrance and a non-parent fragrance include differences in chemical make-up. Typically, a non-parent fragrance is encapsulated within a material before inclusion into a composition and/or article.

It has been surprisingly discovered that, in anhydrous compositions with microcapsules, if too much non-volatile oil is included in the composition, then the presence of the non-volatile oils may degrade the performance of the microcapsules. Without wishing to be bound by this theory, it is believed that the non-volatile oils tend to coat the shells of the microcapsules. The coated microcapsules tend to experience lower frictional forces during mechanical interactions such that the shells of the coated microcapsules are less likely to rupture. As a result, the coated microcapsules may form a less effective delivery system because the coated microcapsules are less likely to release their core material.

Without wishing to be bound by this theory, it is also believed that the non-volatile oils tend to suppress the release of volatile components contained encapsulated by the shells of the microcapsules. Once the shells of the microcapsules rupture, the non-volatile oils in the composition tend to interact with the volatile components released from the microcapsules. This interaction may make the volatile components less likely to escape into the surrounding atmosphere. As a result, the coated microcapsules may form a less effective delivery system.

When an anhydrous composition includes microcapsules, the percentage of non-volatile oils in the composition can be limited to certain ranges, which provide enough oil to still offer product benefits, but not so much that that the oil substantially degrades the performance of the microcapsules. As an example, an anhydrous composition can include microcapsules and non-volatile oils, which form 10-23% of a total mass of the composition. The anhydrous compositions described herein may include from 10% to 15%, or from 10% to 20% by total mass of the composition of non-volatile oils. Although some microcapsules can encapsulate non-volatile oils, it is understood that encapsulated non-volatile oils are not to be included within the percentages mentioned above. In this regard, it is believed that encapsulated non-volatile oils may behave differently from non-encapsulated non-volatile oils when in a composition containing microcapsules.

Where the composition is an invisible solid (e.g. antiperspirant or deodorant), the degree of the benefit provided by the non-volatile oils in the composition can be measured using the Residue Evaluation Test Method, provided herein. The Residue Evaluation Test measures the amount of visible, white residue left after using a composition. When the composition is measured according to the Residue Evaluation Test Method, a composition may have a change in color lightness value of 2.0-6.0, or any incremental value expressed in 0.1 in this range, or any range formed by any of these values for change in color lightness. As an example, when measured according to the Residue Evaluation Test Method, a composition may have a change in color lightness value of 2.0-4.0. The higher the score on the Residue Evaluation Test Method, the more visible white residue is left after applying the composition. The benefit of reducing the level of non-volatiles in compositions including microcapsules is illustrated in Table 2 included herein.

The compositions include microcapsules. The microcapsules comprise a core material and a shell encapsulating the core material and the shell includes a polyacrylate material.

The microcapsules may be friable microcapsules. A friable microcapsule is configured to release the core material when the outer shell is ruptured. The rupture can be caused by forces applied to the shell during mechanical interactions. Some or all of the microcapsules can have various fracture strengths. For at least a first group of the provided microcapsules, the microcapsule can have a shell with a fracture strength of 0.2-10.0 mega Pascals, when measured according to the Fracture Strength Test Method, or any incremental value expressed in 0.1 mega Pascals in this range, or any range formed by any of these values for fracture strength. As an example, a microcapsule can have a shell with a fracture strength of 0.2-2.0 mega Pascals.

Some or all of the microcapsules can be made by interfacial polymerization. An interfacial polymerization process may be utilized to manufacture microcapsules, such a process may include adding one or more reactants to an oil phase and one or more reactants to an aqueous phase. These phases are brought together, typically in an emulsification process, and the reactants diffuse to the oil/water interface to form products that serve as the building blocks for the shell. The continuous diffusion of the reactants in the two phases to the interface, and the subsequent reaction of monomers to form polymers, may lead to the formation of the microcapsule. The microcapsules are spray-dried to reduce the moisture associated with the microcapsules before inclusion in to a composition like an anhydrous composition.

Some or all of the microcapsules can have various core to shell ratios. For at least a first group of the provided microcapsules, each microcapsule can have a shell, a core material within the shell, and a core to shell mass ratio that is greater than or equal to: 70% to 30%, 75% to 25%, 80% to 20%, 85% to 15%, 90% to 10%, 95% to 5%.

In some examples, the microcapsule's shell comprises a reaction product of a first mixture in the presence of a second mixture comprising an emulsifier, the first mixture comprising a reaction product of i) an oil soluble or dispersible amine with ii) a multifunctional acrylate or methacrylate monomer or oligomer, an oil soluble acid and an initiator, the

emulsifier comprising a water soluble or water dispersible acrylic acid alkyl acid copolymer, an alkali or alkali salt, and optionally a water phase initiator. In some examples, said amine is an aminoalkyl acrylate or aminoalkyl methacrylate.

In some examples, the microcapsules include a core material and a shell surrounding the core material, wherein the shell comprises: a plurality of amine monomers selected from the group consisting of aminoalkyl acrylates, alkyl aminoalkyl acrylates, dialkyl aminoalykl acrylates, aminoalkyl methacrylates, alkylamino aminoalkyl methacrylates, dialkyl aminoalykl methacrylates, tertiarybutyl aminethyl methacrylates, diethylaminoethyl methacrylates, dimethylaminoethyl methacrylates, dipropylaminoethyl methacrylates, and mixtures thereof; and a plurality of multifunctional monomers or multifunctional oligomers.

Some or all of the microcapsules can have shells made in any size, shape, and configuration known in the art. The shells include a polyacyrylate material, such as a polyacrylate random copolymer. For example, the polyacrylate random copolymer can have a total polyacrylate mass, which includes ingredients selected from the group including: amine content of 0.2-2.0% of total polyacrylate mass; carboxylic acid of 0.6-6.0% of total polyacrylate mass; and a combination of amine content of 0.1-1.0% and carboxylic acid of 0.3-3.0% of total polyacrylate mass.

When a microcapsule's shell includes a polyacrylate material, and the shell has an overall mass, the polyacrylate material can form 5-100% of the overall mass, or any integer value for percentage in this range, or any range formed by any of these values for percentage. As examples, the polyacrylate material can form at least 5%, at least 10%, at least 25%, at least 33%, at least 50%, at least 70%, or at least 90% of the overall mass.

Some or all of the microcapsules can have various shell thicknesses. For at least a first group of the provided microcapsules, the microcapsule can have a shell with an overall thickness of 1-300 nanometers, or any integer value for nanometers in this range, or any range formed by any of these values for thickness. As an example, microcapsules can have a shell with an overall thickness of 2-200 nanometers.

The composition is an anhydrous composition and may include microcapsules wherein, for at least a first group of the microcapsules, the microcapsules encapsulates one or more benefit agents. The benefit agents(s) can be in the form of solids and/or liquids. The benefit agent(s) can include one or more of chromogens, dyes, fragrances, flavorants, sweeteners, oils, pigments, pharmaceuticals, moldicides, herbicides, fertilizers, phase change materials, warming sensates, cooling sensates, antimicrobial agents, adhesives, and any other kind of benefit agent known in the art, in any combination.

The composition also contains one or more additional delivery systems for providing one or more benefit agents, in addition to the microcapsules. The additional delivery system(s) can differ in kind from the microcapsules. For example, wherein the microcapsules encapsulate a fragrance, the additional delivery system is a moisture-triggered delivery system fragrance delivery system, such as a cyclic oligosachharide, starch, starch-derivative, polysaccharide-based encapsulation system, and combinations thereof. The compositions can also include a parent fragrance dispersed throughout the composition.

Where the composition contains microcapsules that encapsulate a liquid fragrance, the efficacy of those microcapsules, in delivering that fragrance, can be measured using the Headspace Test Method, provided herein. A composition can have an increase in headspace value of 1,000 - 15,000%, when measured according to the Headspace Test Method or any integer value for percentage in this range, or any range formed by any of these values for percentage. As an example, a composition can have an increase in headspace value of 4,000 - 10,000%, when measured according to the Headspace Test Method

The composition may include various amounts of one or more non-volatile oils, in various proportions, which altogether can form a percent mass of the total mass of the composition. Total mass is defined as a composition containing various ingredients that sum to 100% as added into said formulation. The term "non-volatile," as used herein, unless otherwise specified, refers to those materials that are liquid under ambient conditions and which have a measurable vapor pressure at 25 degrees C. These materials typically have a vapor pressure less than 0.01 mmHg, and an average boiling point typically greater than 250° C.

As an example, various amounts of one or more non-volatile oils, in various proportions altogether can form a percent mass of the total mass of the composition, wherein the percent mass is 10-15%. As further examples, various amounts of one or more non-volatile oils, in various proportions altogether can form a percent mass of the total mass of the composition, wherein the percent mass is from 3% to 20%, or from 3% to 18%, or from 3% to 15%. Non-limiting examples of non-volatile oils include emollients, petrolatum, reside maskers, mineral oils, dimethicone, C12-15 akyl benzoate, PPG-14 butyl ether, phenyl trimethicone, isopropyl myristate, 2-phenyl ethyl benzoate, and any other kind of non-volatile oil known in the art, in any combination. Emollients are referred to as a material that can provide the formulation with softening properties and give the product a smooth feel and appearance on the skin. Residue Maskers are defined as non-volatile oils having a refractive index similar to that of the antiperspirant active; typically, these materials will have a refractive index of 1.375 to 1.5.

The composition can include various amounts of one or more volatile oils, in various proportions, which altogether can form a percent mass of the total mass of the composition, wherein the percent mass is 10-65% or any integer value for percentage in this range, or any range formed by any of these values for percentage. As an example, the composition

can have volatile oils, which form 20-40% of the total mass of the composition. The term "volatile," as used herein, unless otherwise specified, refers to those materials that are liquid under ambient conditions and which have a measurable vapor pressure at 25° C. These materials typically have a vapor pressure greater than 0.01 mmHg, more typically from 0.02 mmHg to 20 mmHg, and an average boiling point typically less than 250° C, more typically less than 235° C.

Thus, when an anhydrous personal care composition includes microcapsules, the percentage of non-volatile oils in the composition can be limited to certain ranges, which provide enough oil to still offer product benefits, but not so much that that the oil substantially degrades the performance of the microcapsules.

Cyclic Oligosaccharides

The compositions or articles described herein may include a moisture-triggered fragrance technology incorporating cyclic oligosaccharides. As used herein, the term "cyclic oligosaccharide" means a cyclic structure comprising six or more saccharide units. The cyclic oligosaccharides can have six, seven, or eight saccharide units or mixtures thereof. It is common in the art to refer to six, seven and eight membered cyclic oligosaccharides as α, β, and γ, respectively. The cyclic oligosaccharides that may be useful include those that are soluble in water, ethanol, or both water and ethanol. The cyclic oligosaccharides useful herein may have a solubility of at least 0.1g/100 ml, at 25° C and 1 atm of pressure in either water, ethanol, or both water and ethanol. The compositions disclosed herein may comprise from 0.001% to 40%, from 0.1% to 25%, from 0.3% to 20%, from 0.5% to 10%, or from 0.75% to 5%, by weight of the composition, of a cyclic oligosaccharide. The compositions disclosed herein may comprise from 0.001% to 40%, from.1% to 25%, from 0.3% to 20%, from 0.5% to 10%, or from 0.75% to 5%, by weight of the composition, of a cyclic oligosaccharide.

The cyclic oligosaccharide may comprise any suitable saccharide or mixture of saccharides. Examples of suitable saccharides include, but are not limited to, glucose, fructose, mannose, galactose, maltose, and mixtures thereof. The cyclic oligosaccharide, or mixture of cyclic oligosaccharides, may be substituted by any suitable substituent or mixture of substituents. Herein the use of the term "mixture of substituents" means that two or more different suitable substituents may be substituted onto one cyclic oligosaccharide. Suitable examples of substituents include, but are not limited to, alkyl groups, hydroxyalkyl groups, dihydroxyalkyl groups, carboxyalkyl groups, aryl groups, maltosyl groups, allyl groups, benzyl groups, alkanoyl groups, and mixtures thereof. These substituents may be saturated or unsaturated, straight or branched chain. For example, the substituents may include saturated and straight chain alkyl groups, hydroxyalkyl groups, and mixtures thereof. The alkyl and hydroxyalkyl substituents, for example, may also be selected from $C_1$-$C_8$ alkyl or hydroxyalkyl groups, alkyl and hydroxyalkyl substituents from $C_1$-$C_6$ alkyl or hydroxyalkyl groups, and alkyl and hydroxyalkyl substituents from $C_1$-$C_4$ alkyl or hydroxyalkyl groups. The alkyl and hydroxyalkyl substituents may be, for example, propyl, ethyl, methyl, and hydroxypropyl.

In addition to the substituents themselves, the cyclic oligosaccharides may have an average degree of substitution of at least 1.6, wherein the term "degree of substitution" means the average number of substituents per saccharide unit. For example, the cyclic oligosaccharides may have an average degree of substitution of less than 2.8 or from 1.7 to 2.0. The average number of substituents may be determined using common Nuclear Magnetic Resonance techniques known in the art. Examples of cyclic oligosaccharides useful herein include cthe cyclodextrins such as methyl-α-cyclodextrins, methyl-β-cyclodextrins, hydroxypropyl-α-cyclodextrins, hydroxypropyl-β-cyclodextrins, and mixtures thereof. The cyclodextrins may be in the form of particles. The cyclodextrins may also be spray-dried and may also be spray-dried particles.

Fragrances

The compositions or articles may comprise fragrances. As used herein, "fragrance" is used to indicate any odoriferous material. Any fragrance that is cosmetically acceptable may be used in the composition. For example, the fragrance may be one that is a liquid at room temperature. Generally, the fragrance(s) may be present at a level from 0.01 % to 40%, from 0.1 % to 25%, from 0.25% to 20%, or from 0.5% to 15%, by weight of the composition.

A wide variety of chemicals are known as fragrances, including aldehydes, ketones, and esters. More commonly, naturally occurring plant and animal oils and exudates comprising complex mixtures of various chemical components are known for use as fragrances. Non-limiting examples of the fragrances useful herein include pro-fragrances such as acetal pro-fragrances, ketal pro-fragrances, ester pro-fragrances, hydrolyzable inorganic-organic pro-fragrances, and mixtures thereof. The fragrances may be released from the pro-fragrances in a number of ways. For example, the fragrance may be released as a result of simple hydrolysis, or by a shift in an equilibrium reaction, or by a pH-change, or by enzymatic release. The fragrances herein may be relatively simple in their chemical make-up, comprising a single chemical, or may comprise highly sophisticated complex mixtures of natural and synthetic chemical components, all chosen to provide any desired odor.

The fragrances may have a boiling point (BP) of 500°C or lower, 400°C or lower, or 350°C or lower. The BP of many fragrances are disclosed in *Perfume and Flavor Chemicals* (Aroma Chemicals), Steffen Arctander (1969). The

ClogP value of the fragrances may be 0.1 or greater, 0.5 or greater, 1.0 or greater, and 1.2 or greater. As used herein, "ClogP" means the logarithm to the base 10 of the octanol/water partition coefficient. The ClogP can be readily calculated from a program called "CLOGP" which is available from Daylight Chemical Information Systems Inc., Irvine Calif., USA. Octanol/water partition coefficients are described in more detail in U.S. Pat. No. 5,578,563.

Suitable fragrances are also disclosed in U.S. Pat. No. 4,145,184, U.S. Pat. No. 4,209,417, U.S. Pat. No. 4,515,705, and U.S. Pat. No. 4,152,272. Non-limiting examples of fragrances include animal fragrances such as musk oil, civet, castoreum, ambergris, plant fragrances such as nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

Other examples of suitable fragrances include, but are not limited to, chemical substances such as acetophenone, adoxal, aldehyde C-12, aldehyde C-14, aldehyde C-18, allyl caprylate, ambroxan, amyl acetate, dimethylindane derivatives, $\alpha$-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, $\alpha$-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-$\alpha$-naphthyl ketone, methylphenylcarbinyl acetate, mugetanol, $\gamma$-nonalactone, octanal, phenyl ethyl acetate, phenylacetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, $\gamma$-undecalactone, undecenal, vanillin, veloutone, verdox, and mixtures thereof.

Method of Use

The compositions described herein may be packaged with any container known in the art or with any dispenser suitable for delivering the composition to a substrate. The composition may be applied to any substance where moisture and/or friction is available to trigger the release of the fragrance. When the composition is applied to the human body, the composition may be applied to any area of the skin or may be applied to any area of the body. The compositions may be used as consumer products (i.e. products intended to be sold to consumers without further modification or processing). Moreover, the microcapsules may be applied to any article, such as a fabric or any absorbent material including, but not limited to, feminine hygiene products, diapers, and adult incontinence products. The composition may also be incorporated into an article.

The compositions and articles described herein may also be used to overcome the habituation experienced by some consumers to a parent fragrance in an article and/or composition. For example, some consumers are known to suffer from habituation to the fragrance expressed by a composition and/or article such that the fragrance becomes less noticeable over time. One method of overcoming habituation in a composition and/or article is to incorporate a non-parent fragrance and a parent fragrance wherein the fragrances are expressed at different times or in an oscillating fashion. However, this is not easily done in practice as simply mixing the parent fragrance and non-parent fragrance together may result in a bloom that is a combination of both types of fragrances such that some consumers may still experience habituation to the combination. Additionally, many encapsulation technologies that allow for a triggered- or delayed-release of a non-parent fragrance result in some level of mixing of the parent and non-parent fragrances.

In this regard, some encapsulation technologies may not effectively prevent diffusion of the encapsulated, non-parent fragrance into the composition and/or the diffusion of the parent fragrance into the core of the encapsulation material such that there is a mixing of the parent fragrance and the non-parent fragrance. Although such technologies may provide a triggered- or delayed-release of a fragrance, these technologies may not be able to overcome the habituation as a result of the mixing of the parent and non-parent fragrances that occurs before the consumer uses the product.

Thus, the technologies described herein may overcome habituation in a composition and/or article by delaying the release of the non-parent fragrance so that the parent fragrance and the non-parent fragrance(s) bloom at different times and such that the non-parent and parent fragrance do not mix in the composition and/or article to a significant degree before usage. The technologies described herein can also be used to combat habituation without the need for a moisture-triggering event. For example, friction alone may be a sufficient triggering event that is used to express the non-parent fragrance. Additionally, the technologies described herein may also be used to combat habituation by utilizing

multiple different classes of triggering events, such as moisture-triggering events and friction-triggered events.

The technologies described herein may also be used to improve the releasability of the core material of microcapsules present in anhydrous compositions. The method can include preparing an anhydrous composition according to claim 1. By reducing the amount of non-volatiles to the levels described herein, the microcapsules included within the anhydrous composition are more likely to release their core material (e.g. fragrances) during use of the anhydrous composition. Thus, the lower levels of non-volatiles helps deliver a sufficient amount of fragrance throughout the period of use for compositions including microcapsules.

Solid Antiperspirant Compositions

Anhydrous compositions, like solid antiperspirant compositions, may require microcapsules with less than 20% water, preferably with less than 5% water. Free water in such anhydrous compositions can lead to the crystallization of the antiperspirant actives which may affect the performance of the composition when used. Spray-drying a slurry of microcapsules before inclusion into a solid antiperspirant composition is one way of reducing the amount of water associated with the microcapsules. Other ways of reducing the moisture content of the microcapsules are known, such as drying the microcapsules in an oven.

Additionally, for at least some friable microcapsules, such microcapsules may be more flexible in environments containing high levels of water. For example, for at least some microcapsules, said microcapsules may not release their core material (e.g. a fragrance) when friction or other mechanical forces are applied in a hyper-hydrated state. By spray-drying said microcapsules before inclusion into the anhydrous composition, said microcapsules may be more likely to rupture and release their core materials.

Solid antiperspirant compositions may include an antiperspirant active suitable for application to human skin. The concentration of the antiperspirant active in the composition should be sufficient to provide the desired enhanced wetness protection. For example, the active may be present in an amount of from 0.1%, 0.5%, 1%, 5%, or 10%; to 60%, 35%, 30%, 25% or 20%, by weight of the composition. These weight percentages are calculated on an anhydrous metal salt basis exclusive of water and any complexing agents such as glycine, glycine salts, or other complexing agents.

An antiperspirant active can include any compound, composition, or other material having antiperspirant activity. Such actives may include astringent metallic salts, especially inorganic and organic salts of aluminum, zirconium and zinc, as well as mixtures thereof. For example, the antiperspirant actives may include zirconium-containing salts or materials, such as zirconyl oxyhalides, zirconyl hydroxyhalides, and mixtures thereof; and/or aluminum-containing salts such as, for example, aluminum halides, aluminum chlorohydrate, aluminum hydroxyhalides, and mixtures thereof.

1. Aluminum Salts

Aluminum salts useful herein can include those that conform to the formula:

$$Al_2(OH)_aCl_b \cdot x\ H_2O$$

wherein a is from 2 to 5; the sum of a and b is about 6; x is from 1 to 6; where a, b, and x may have non-integer values. For example, aluminum chlorohydroxides referred to as "5/6 basic chlorohydroxide," wherein a is about 5 and "2/3 basic chlorohydroxide", wherein a=4 may be used.

2. Zirconium Salts

Zirconium salts useful herein can include those which conform to the formula:

$$ZrO(OH)_{2-a}Cl_a \cdot x\ H_2O$$

wherein a is from 1.5 to 1.87; x is from 1 to 7; and wherein a and x may both have non-integer values. Useful are zirconium salt complexes that additionally contain aluminum and glycine, commonly known as "ZAG complexes". These complexes can contain aluminum chlorohydroxide and zirconyl hydroxy chloride conforming to the above-described formulas. Examples of two such complexes include aluminum zirconium trichlorohydrex and aluminum zirconium tetrachlorohydrex.

Antiperspirant compositions can also include a structurant to help provide the composition with the desired viscosity, rheology, texture and/or product hardness, or to otherwise help suspend any dispersed solids or liquids within the composition. The term "structurant" may include any material known or otherwise effective in providing suspending, gelling, viscosifying, solidifying, or thickening properties to the composition or which otherwise provide structure to the final product form. These structurants may include, for example, gelling agents, polymeric or nonpolymeric agents, inorganic thickening agents, or viscosifying agents. The thickening agents may include, for example, organic solids,

silicone solids, crystalline or other gellants, inorganic particulates such as clays or silicas, or combinations thereof.

The concentration and type of the structurant selected for use in the antiperspirant composition will vary depending upon the desired product form, viscosity, and hardness. The structurant suitable for use herein, may have a concentration range from 0.1%, 2%, 3%, 5%; or 10%; to 35%, 20%, 10%, or 8%, by weight of the composition. Soft solids will often contain a lower amount of structurant than solid compositions. For example, a soft solid may contain from 1.0% to 9%, by weight of the composition, while a solid composition may contain from 15% to 25%, by weight of the composition, of structurant. This is not a hard and fast rule, however, as a soft solid product with a higher structurant value can be formed by, for example, shearing the product as it is dispensed from a package.

Non-limiting examples of suitable gelling agents include fatty acid gellants, salts of fatty acids, hydroxyl acids, hydroxyl acid gellants, esters and amides of fatty acid or hydroxyl fatty acid gellants, cholesterolic materials, dibenzylidene alditols, lanolinolic materials, fatty alcohols, triglycerides, sucrose esters such as SEFA behenate, inorganic materials such as clays or silicas, other amide or polyamide gellants, and mixtures thereof.

Suitable gelling agents include fatty acid gellants such as fatty acid and hydroxyl or alpha hydroxyl fatty acids, having from 10 to 40 carbon atoms, and ester and amides of such gelling agents. Non-limiting examples of such gelling agents include, but are not limited to, 12-hydroxystearic acid, 12-hydroxylauric acid, 16-hydroxyhexadecanoic acid, behenic acid, eurcic acid, stearic acid, caprylic acid, lauric acid, isostearic acid, and combinations thereof. Preferred gelling agents are 12-hydroxystearic acid, esters of 12-hydroxystearic acid, amides of 12-hydroxystearic acid and combinations thereof.

Other suitable gelling agents include amide gellants such as di-substituted or branched monoamide gellants, monsubstituted or branched diamide gellants, triamide gellants, and combinations thereof, including n-acyl amino acid derivatives such as n-acyl amino acid amides, n-acyl amino acid esters prepared from glutamic acid, lysine, glutamine, aspartic acid, and combinations thereof.

Still other examples of suitable gelling agents include fatty alcohols having at least 8 carbon atoms, at least 12 carbon atoms but no more than 40 carbon atoms, no more than 30 carbon atoms, or no more than 18 carbon atoms. For example, fatty alcohols include but are not limited to cetyl alcohol, myristyl alcohol, stearyl alcohol and combinations thereof.

Non-limiting examples of suitable tryiglyceride gellants include tristearin, hydrogenated vegetable oil, trihydroxysterin (Thixcin® R, available from Rheox, Inc.), rape seed oil, castor wax, fish oils, tripalmitin, Syncrowax® HRC and Syncrowax® HGL-C (Syncrowax® available from Croda, Inc.).

Other suitable structurants include waxes or wax-like materials having a melt point of above 65°C, more typically from 65°C to 130°C, examples of which include, but are not limited to, waxes such as beeswax, carnauba, bayberry, candelilla, montan, ozokerite, ceresin, hydrogenated castor oil (castor wax), synthetic waxes and microcrystalline waxes. Castor wax is preferred within this group. The synthetic wax may be, for example, a polyethylene, a polymethylene, or a combination thereof. Some suitable polymethylenes may have a melting point from 65°C to 75°C. Examples of suitable polyethylenes include those with a melting point from 60°C to 95°C.

Further structurants for use in the solid antiperspirant compositions of the present invention may include inorganic particulate thickening agents such as clays and colloidal pyrogenic silica pigments. For example, colloidal pyrogenic silica pigments such as Cab-O-Sil®, a submicroscopic particulated pyrogenic silica may be used. Other known or otherwise effective inorganic particulate thickening agents that are commonly used in the art can also be used in the solid antiperspirant compositions of the present invention. Concentrations of particulate thickening agents may range, for example, from 0.1%, 1%, or 5%; to 35%, 15%, 10% or 8%, by weight of the composition.

Suitable clay structurants include montmorillonite clays, examples of which include bentonites, hectorites, and colloidal magnesium aluminum silicates. These and other suitable clays may be hydrophobically treated, and when so treated will generally be used in combination with a clay activator. Non-limiting examples of suitable clay activators include propylene carbonate, ethanol, and combinations thereof. When clay activators are present, the amount of clay activator will typically range from 40%, 25%, or 15%; to 75%, 60%, or 50%, by weight of the clay.

Solid antiperspirant compositions may further include anhydrous liquid carriers. These are present, for example, at concentrations ranging from 10%, 15%, 20%, 25%; to 99%, 70%, 60%, or 50%, by weight of the composition. Such concentrations will vary depending upon variables such as product form, desired product hardness, and selection of other ingredients in the composition. The anhydrous carrier may be any anhydrous carrier known for use in personal care applications or otherwise suitable for topical application to the skin. For example, anhydrous carriers may include, but are not limited to volatile and nonvolatile fluids.

An antiperspirant composition may further include a volatile fluid such as a volatile silicone carrier. Volatile fluids are present, for example, at concentrations ranging from 20% or from 30%; to 80%, or no 60%, by weight of the composition. The volatile silicone of the solvent may be cyclic, linear, and/or branched chain silicone. "Volatile silicone", as used herein, refers to those silicone materials that have measurable vapor pressure under ambient conditions.

The volatile silicone may be a cyclic silicone. The cyclic silicone may have from 3 silicone atoms, or from 5 silicone atoms; to 7 silicone atoms, or 6 silicone atoms. For example, volatile silicones may be used which conform to

the formula:

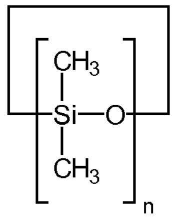

wherein n is from 3, or from 5; to 7, or 6. These volatile cyclic silicones generally have a viscosity of less than 10 centistokes at 25 °C. Suitable volatile silicones for use herein include, but are not limited to, Cyclomethicone D5 (commercially available from G. E. Silicones); Dow Corning 344, and Dow Corning 345 (commercially available from Dow Corning Corp.); and GE 7207, GE 7158 and Silicone Fluids SF-1202 and SF-1173 (available from General Electric Co.). SWS-03314, SWS-03400, F-222, F-223, F-250, F-251 (available from SWS Silicones Corp.); Volatile Silicones 7158, 7207, 7349 (available from Union Carbide); Masil SF-V (available from Mazer) and combinations thereof.

An antiperspirant composition may further comprise a non-volatile fluid. These non-volatile fluids may be either non-volatile organic fluids or non-volatile silicone fluids. The non-volatile organic fluid can be present, for example, at concentrations ranging from 1%, from 2%; to 20%, or 15%, by weight of the composition.

Non-limiting examples of nonvolatile organic fluids include, but are not limited to, mineral oil, PPG-14 butyl ether, isopropyl myristate, petrolatum, butyl stearate, cetyl octanoate, butyl myristate, myristyl myristate, C12-15 alkylbenzoate (e.g., Finsolv.TM.), dipropylene glycol dibenzoate, PPG-15 stearyl ether benzoate and blends thereof (e.g. Finsolv TPP), neopentyl glycol diheptanoate (e.g. Lexfeel 7 supplied by Inolex), octyldodecanol, isostearyl isostearate, octododecyl benzoate, isostearyl lactate, isostearyl palmitate, isononyl/ isononoate, isoeicosane, octyldodecyl neopentanate, hydrogenated polyisobutane, and isobutyl stearate.

An antiperspirant composition may further include a non-volatile silicone fluid. The non-volatile silicone fluid may be a liquid at or below human skin temperature, or otherwise in liquid form within the anhydrous antiperspirant composition during or shortly after topical application. The concentration of the non-volatile silicone may be from 1%, from 2%; to 15%, 10%, by weight of the composition. Nonvolatile silicone fluids of the present invention may include those which conform to the formula:

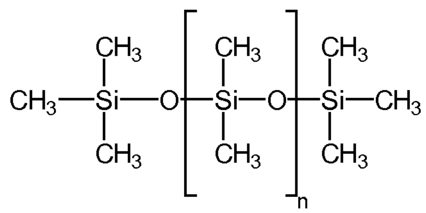

wherein n is greater than or equal to 1. These linear silicone materials may generally have viscosity values of from 5 centistokes, from 10 centistokes; to 100,000 centistokes, 500 centistokes, 200 centistokes, or 50 centistokes (from $5.10^{-6}$ $m^2/s$, from $10.10^{-6}$ $m^2/s$ ; to 0,1 $m^2/s$, 0,5 $m^2/s$, 0,002 $m^2/s$, $5.10^{-5} m^2/s$), as measured under ambient conditions.

Specific non limiting examples of suitable nonvolatile silicone fluids include Dow Corning 200, hexamethyldisiloxane, Dow Corning 225, Dow Corning 1732, Dow Coming 5732, Dow Coming 5750 (available from Dow Corning Corp.); and SF-96, SF-1066 and SF18(350) Silicone Fluids (available from G.E. Silicones).

Low surface tension non-volatile solvent may be also be used. Such solvents may be selected from the group consisting of dimethicones, dimethicone copolyols, phenyl trimethicones, alkyl dimethicones, alkyl methicones, and mixtures thereof. Low surface tension non-volatile solvents are also described in U.S. Pat. No. 6,835,373 (Kolodzik et al.).

An antiperspirant composition may include a malodor reducing agent. Malodor reducing agents include components other than the antiperspirant active within the composition that act to eliminate the effect that body odor has on fragrance display. These agents may combine with the offensive body odor so that they are not detectable including, but not limited to, suppressing evaporation of malodor from the body, absorbing sweat or malodor, masking the malodor or microbiological activity on odor causing organisms. The concentration of the malodor reducing agent within the composition is sufficient to provide such chemical or biological means for reducing or eliminating body odor. Although the concentration will vary depending on the agent used, generally, the malodor reducing agent may be included within the composition from 0.05%, 0.5%, or 1%; to 15%, 10%, or 6%, by weight of the composition.

Malodor reducing agents may include, but are not limited to, pantothenic acid and its derivatives, petrolatum, menthyl acetate, uncomplexed cyclodextrins and derivatives thereof, talc, silica and mixtures thereof.

For example, if panthenyl triacetate is used, the concentration of the malodor reducing agent may be from 0.1% or 0.25%; to 3.0%, or 2.0%, by weight of the composition. Another example of a malodor reducing agent is petrolatum which may be included from 0.10%, or 0.5%; to 15%, or 10%, by weight of the composition. A combination may also be used as the malodor reducing agent including, but not limited to, panthenyl triacetate and petrolatum at levels from 0.1%, or 0.5%; to 3.0 %, or 10%, by weight of the composition. Menthyl acetate, a derivative of menthol that does not have a cooling effect, may be included from 0.05%, or 0.01%; to 2.0%, or 1.0%, by weight of the composition. The malodor reducing agent may be in the form of a liquid or a semi-solid such that it does not contribute to product residue.

Sensory Test Method

Blinded, monadic, single-use evaluations are conducted under controlled environmental conditions, by a trained expert panel using standardized procedures. Approximately 10-20 trained panelists participate in each evaluation. Evaluations are done on the underarms. Product applications are randomized across the panel for left and right sides and for product used first and used second. The specific sensory attributes evaluated for each test are selected prior to test placement. Panelists are screened, selected and trained to do visual, tactile, and odor intensity evaluation. Panelists wash and dry the area to be used in evaluation.

The primary product evaluation is conducted on the underarm. 0.40 ± 0.02g of the first test product is applied to the right underarm. This application procedure is repeated for the second test product in the left underarm. Product characteristics (visual, tactile and fragrance intensity) are evaluated and rated at various intervals up to 18 hours following application. In order to assess release of fragrance via the microcapsules, panelists are instructed to move the underarm back and forth ensuring that there is skin to skin contact to generate friction in the underarm. Perform this motion 5 complete times (10 back and forth motions) prior to evaluating fragrance intensity in the underarm.

Visual assessments are made with the aid of a mirror. Panelists evaluate no more than two products per test site per day. Each test site is washed and equilibrated between test products.

Panelists ratings for all products in a study are combined. Mean intensity scores are calculated and a one-way ANCOVA on products adjusted for weight with Tukey post-hoc testing when appropriate.

Fracture Strength Test Method

One skilled in the art will recognize that various protocols may be constructed for the extraction and isolation of microcapsules from finished products, and will recognize that such methods require validation via a comparison of the resulting measured values, as measured before and after the microcapsules' addition to and extraction from the finished product. The isolated microcapsules are then formulated in de-ionized (DI) water to form a slurry for characterization.

To calculate the percentage of microcapsules which fall within a claimed range of fracture strengths, three different measurements are made and two resulting graphs are utilized. The three separate measurements are namely: i) the volume-weighted particle size distribution (PSD) of the microcapsules; ii) the diameter of at least 10 individual microcapsules within each of 3 specified size ranges, and; iii) the rupture-force of those same 30 or more individual microcapsules. The two graphs created are namely: a plot of the volume-weighted particle size distribution data collected at i) above; and a plot of the modeled distribution of the relationship between microcapsule diameter and fracture-strength, derived from the data collected at ii) and iii) above. The modeled relationship plot enables the microcapsules within a claimed strength range to be identified as a specific region under the volume-weighted PSD curve, and then calculated as a percentage of the total area under the curve.

a.) The volume-weighted particle size distribution (PSD) of the microcapsules is determined via single-particle optical sensing (SPOS), also called optical particle counting (OPC), using the AccuSizer 780 AD instrument, or equivalent, and the accompanying software CW788 version 1.82 (Particle Sizing Systems, Santa Barbara, California, U.S.A.). The instrument is configured with the following conditions and selections: Flow Rate = 1 ml / sec; Lower Size Threshold = 0.50 μm; Sensor Model Number = LE400-05SE; Autodilution = On; Collection time = 120 sec; Number channels = 512; Vessel fluid volume = 50ml; Max coincidence = 9200. The measurement is initiated by putting the sensor into a cold state by flushing with water until background counts are less than 100. A capsule slurry, and its density of particles is adjusted with DI water as necessary via autodilution to result in particle counts of at least 9200 per ml. During a time period of 120 seconds the suspension is analyzed. The resulting volume-weighted PSD data are plotted and recorded, and the values of the mean, $5^{th}$ percentile, and $90^{th}$ percentile are determined.

b.) The diameter and the rupture-force value (also known as the bursting-force value) of individual microcapsules are measured via a computer-controlled micromanipulation instrument system which possesses lenses and cameras able to image the microcapsules, and which possesses a fine, flat-ended probe connected to a force-transducer (such as the Model 403A available from Aurora Scientific Inc, Canada, or equivalent), as described in: Zhang, Z. et

al. (1999) "Mechanical strength of single microcapsules determined by a novel micromanipulation technique." J. Microencapsulation, vol 16, no. 1, pages 117-124, and in: Sun, G. and Zhang, Z. (2001) "Mechanical Properties of Melamine-Formaldehyde microcapsules." J. Microencapsulation, vol 18, no. 5, pages 593-602, and as available at the University of Birmingham, Edgbaston, Birmingham, UK.

c.) A drop of the microcapsule suspension is placed onto a glass microscope slide, and dried under ambient conditions for several minutes to remove the water and achieve a sparse, single layer of solitary particles on the dry slide. Adjust the concentration of microcapsules in the suspension as needed to achieve a suitable particle density on the slide. More than one slide preparation may be needed.

d.) The slide is then placed on a sample-holding stage of the micromanipulation instrument. Thirty or more microcapsules on the slide(s) are selected for measurement, such that there are at least ten microcapsules selected within each of three pre-determined size bands. Each size band refers to the diameter of the microcapsules as derived from the Accusizer-generated volume-weighted PSD. The three size bands of particles are: the Mean Diameter +/- 2 $\mu$m; the $5^{th}$ Percentile Diameter +/- 2 $\mu$m; and the $90^{th}$ Percentile Diameter +/- 2 $\mu$m. Microcapsules which appear deflated, leaking or damaged are excluded from the selection process and are not measured.

e.) For each of the 30 or more selected microcapsules, the diameter of the microcapsule is measured from the image on the micromanipulator and recorded. That same microcapsule is then compressed between two flat surfaces, namely the flat-ended force probe and the glass microscope slide, at a speed of 2 $\mu$m per second, until the microcapsule is ruptured. During the compression step, the probe force is continuously measured and recorded by the data acquisition system of the micromanipulation instrument.

f.) The cross-sectional area is calculated for each of the microcapsules, using the diameter measured and assuming a spherical particle ($\pi r^2$, where r is the radius of the particle before compression). The rupture force is determined for each sample by reviewing the recorded force probe measurements. The measurement probe measures the force as a function of distance compressed. At one compression, the microcapsule ruptures and the measured force will abruptly stop. This maxima in the measured force is the rupture force.

g.) The Fracture Strength of each of the 30 or more microcapsules is calculated by dividing the rupture force (in Newtons) by the calculated cross-sectional area of the respective microcapsule.

h.) On a plot of microcapsule diameter versus fracture-strength, a Power Regression trend-line is fit against all 30 or more raw data points, to create a modeled distribution of the relationship between microcapsule diameter and fracture-strength.

i.) The percentage of microcapsules which have a fracture strength value within a specific strength range is determined by viewing the modeled relationship plot to locate where the curve intersects the relevant fracture-strength limits, then reading off the microcapsule size limits corresponding with those strength limits. These microcapsule size limits are then located on the volume-weighted PSD plot and thus identify an area under the PSD curve which corresponds to the portion of microcapsules falling within the specified strength range.

The identified area under the PSD curve is then calculated as a percentage of the total area under the PSD curve. This percentage indicates the percentage of microcapsules falling with the specified range of fracture strengths.

Headspace Test Method

Sample Preparation

1. For each composition to be tested, prepare 3 Professional Aerosol Testing cardboard blotter cards of 7.6 x 12.7 cm size, as supplied by Orlandi Inc. (Farmingdale, New York, USA), , plus 1 additional blotter card for zNose calibration (see next section). Between 0.23 - 0.27 g of finished product composition is applied to the blotter cards for sampling.
Before applying finished product to the blotter cards, prepare or prime the dispensing device according to package directions. For a cream/conditioning/semi-solid product, expose the product until finished product is seen coming through all dispensing holes in the devices' application surface, then wipe the application surface clean with paper towel. For an invisible solid product, expose the product until the top rounded dome of the stick is fully exposed and then remove the exposed dome from the stick with a cutting wire by sliding across top of packaging, to achieve a flat surface on the stick of product.
2. Pre-weigh each blotter card with an analytical balance. For cream/conditioning/semi-solid products/fluid antiperspirants/body powders/foot powders/aerosol antiperspirants, apply/spray (aerosols) the composition evenly to the inner part of the blotter (leaving a 1.3 cm wide zone without product around the outside edge of the blotter card). Continue applying until between 0.23 - 0.27 g of composition is applied, using a balance to determine the weight. For invisible solid product, expose the cleanly cut stick surface until approximately 0.3 cm of the finished product is exposed above the packaging material, then apply the composition evenly in a circular motion to the inner part of the blotter card, leaving

a 1.3 cm wide zone without product around the outside edge of the blotter card. Continue applying until 0.23 - 0.27g of composition is applied, using the balance to determine the weight. If the product composition does not appear evenly distributed across the application area upon visual evaluation, dispose of the blotter card and repeat the application process with a new card.

3. Repeat steps 1 and2 for each product composition to be sampled.

4. Once 3 blotter cards have been prepared for each composition to be sampled, lay the cards out on paper towels with finished product side exposed for a period of 4-6 hours before conducting the zNose evaluation

5. After the drydown period, roll each blotter card into a cylinder shape across the long axis of the card and put into a 207 mL clear polyethyle terephthalate disposable beverage cup with lid, such as available from Solo Cup Company (Lake Forest, Illinois, USA) . Arrange the card so the finished product side of the blotter is facing the inside of the cup. Close the lid. Repeat for all blotter cards. Samples are now prepped and in a controlled headspace ready for evaluation zNose evaluation

1. Prepare the 7100 Benchtop zNose Fast-GC Analyzer (Model # MEA007100 with MicroSenseESTCal System Software version 5.44.28) with DB-624 column, as available from Electronic Sensor Technology Inc. (Newbury Park, California, USA), or equivalent, for evaluations, as defined in manufacturer's instructions.

2. Turn on zNose and perform daily cleaning steps. zNose is clean and operational when all 'peaks' are below 100 counts per mfr instructions..

3. Ensure 'Test Settings' are set according to the following:

| | | |
|---|---|---|
| a. | Sensor: | 70 °C |
| b. | Column: | 40 °C |
| c. | Valve: | 145 °C |
| d. | Inlet: | 200 °C |
| e. | Trap: | 200 °C |
| f. | Pump Time: | 5 seconds |

4. Once test settings match, calibrate the zNose with n-alkanes standard. This will ensure zNose is operating according to manufacturer standard.

5. Once cleaned and calibrated, prepare to run the 'additional' blotters from Step#1. Run samples according to manufacturer instructions. Once all additional blotters have been run, create a new alarm file. The new alarm file will contain no tagged peaks. Tag all non-fragrance peaks from all of the additional blotters run according to manufacturer instructions. All non-fragrance peaks are now identified for the testing the following testing

6. Sampling order should be selected at random from all the samples to be tested.

7. Each sample cup is analyzed on the zNose three times according to the manufacturer's instructions, with a cleaning step between each run by bubbling methanol for 5 seconds, followed by a blank sample. If peaks remain in the results from the blank sample, increase the amount of time for bubbling methanol until no peaks remain. When no peaks remain, run the first sample a second time. Continue to run and clean between runs until 3 triplicate runs have been completed per composition. This is the 'prerub' evaluation data.

8. Repeat steps 6 and 7 for all cups to be tested for prerub evaluations (3 runs per composition). All analytical testing should take place within a 1 hour window 4-6 hours after application of the composition to the blotter cards).

9. After prerub evaluations, remove blotters from the cups then fold the cards in half with the finished product application side on the inside. Using both hands, rub the outside of the folded card with the force required to break an egg, using a back and forth motion ten times, to cover the whole of one side of the folded card. Return the cards to their respective cups, and reseal.

10. Repeat steps 6 - 8 for all cups. This is the 'postrub' evaluation. Once all cups have been tested, transfer all data to a spreadsheet and sum the total area under all peaks associated with the fragrance.

11. For each cup tested, the triplicate analyses will result in 3 'total peak area' measurements for the prerub condition, and another 3 such values for the postrub condition. For each cup, calculate the average, standard deviation, and %Relative Standard Deviation (%RSD) of the 3 total peak area values, separately for the prerub and postrub evaluations, so that each cup is represented by a single prerub and a single postrub total peak area value 12. Repeat for all cups tested. Identify the data that represents each of the 3 cups prepared per composition tested. For each composition, calculate the Mean, standard deviation, and %RSD of the total peak area values from these 3 cups, separately for the prerub and postrub evaluations, such that each composition is represented by a single prerub and a single postrub Mean Total Peak Area, with associated standard deviation and %RSD. Calculate the Percentage Increase in Headspace Value by comparing the Mean Total Peak Area (MTPA) for each composition, postrub versus prerub, relative to the prerub value, as per the following equation:

$$\% \text{ Increase in Headspace Value} = ( (\text{Postrub MTPA} - \text{Prerub MTPA}) / \text{Prerub MTPA}) \times 100$$

13. At least 3 separate samples (eg., 3 deodorant sticks) are measured per composition. Ideally, these samples will represent at least 3 different manufacturing lots or production batches.

Residue Evaluation Test Method

1. Prepare 3 swatches of a black vinyl fabric (such as PT514189 Softside black vinyl available from Proquinal S.A, in Bogotá D.C., Colombia) for each composition to be measured, plus 1 additional swatch. Each swatch should be cut to be approximately 23 x 9 cm in size.
2. Before applying finished product to the swatches, prepare or prime the dispensing device according to package directions. For a cream/conditioning/semi-solid product, expose the product until finished product is seen coming through all dispensing holes in the devices' application surface, then wipe the application surface clean with paper towel. For an invisible solid product, expose the product until the top rounded dome of the stick is fully exposed and then remove the exposed dome from the stick with a cutting wire by sliding across top of packaging, to achieve a flat surface on the stick of product.
3. Pre-weigh each swatch with an analytical balance. For cream/conditioning/semi-solid/powder products, apply the composition evenly to the inner part of the swatch (leaving a 1.9 cm wide zone without product around the outside edge of the swatch). Continue applying until between 0.18 - 0.22 g of composition is applied, using balance to determine the weight. For invisible solid product, expose the cleanly cut stick surface until approximately 0.3 cm of the finished product is exposed above the packaging material, then apply the composition evenly in a circular motion to the inner part of the swatch, leaving a 1.9 cm wide zone without product around the outside edge of the swatch. Continue applying until 0.18 - 0.22 g of composition is applied, using balance to determine the weight. If the product composition does not appear evenly distributed across the application area upon visual evaluation, dispose of the swatch and repeat the application process with a new swatch.
4. Once the composition has been applied to all 3 swatches, analyze them by color measurement using a CR-300 Chroma Meter with the accompanying Data Processor DP-301 software (Konica Minolta Sensing Americas Inc., Ramsey, New Jersey, USA), or equivalent. This is a tri-stimulus colorimeter instrument which measures reflected light across an 8 mm diameter measuring area via a diffuse illumination/0° viewing geometry with the specular component included. It utilizes a pulsed xenon arc lamp. The color values are reported in the CIE 1976 L*a*b* color space, and calculated using a 10 degree observer and type D65 standard illuminant. Follow the manufacturer's directions for each measurement. For each measurement, take a reading on an area of the swatch where the composition was applied. The Chroma Meter will display an L*a*b* value for each measurement. Record this value. Repeat until 5 measurements have been taken on the first swatch. Immediately repeat with the other 2 swatches, for total of 15 readings (5 per swatch). All 15 readings per composition should be recorded within 5 minutes after application of the composition to swatches.
5. Repeat steps 1-4 for each composition to be measured.
6. Using the additional swatch from Step#1 which has no product composition applied to it, use the Chroma Meter to acquire a 'baseline read'. For the baseline reads, acquire 5 nonoverlapping measurements of that undosed swatch. Record the L*a*b* values.
7. Once all compositions are evaluated, compile the data. The 'L*' value from the L*a*b* data is of concern. For each composition, calculate the mean of the 15 'L*' values (5 per swatch). This is the 'test mean' for that composition. Calculate the mean of the 'L*' values from the 5 L*a*b* measurements from the baseline reads in Step#6. This is the 'baseline mean'. For each composition, subtract the baseline mean L* from that composition's test mean L*. This differential represents ΔL* which is the change in color lightness value, for each respective composition.
8. A higher ΔL* score indicates a higher change in color lightness value, indicating greater residue.

Examples

Following, in Table 1, are Examples of antiperspirant compositions. Examples A, B, and C are invisible solid anhydrous antiperspirant compositions including microcapsules made by interfacial polymerization and subsequently dried by a spray-drying process, wherein the microcapsules encapsulate a perfume and varying percentages of non-volatile oils.

Formulation examples A through C and G through H were prepared by conventional mixing technique, adding all of the raw materials (except Aluminum Zirconium Trichlorohydrex Glycine Powder, perfume, and polyacrylate microcapsule) to a mix tank, heating it to a temperature of 80 °C to melt the structurants and other higher melt point ingredients, and holding it at that temperature until the ingredients are melted. At this point, the batch is cooled to 70-75°C and the Aluminum Zirconium Trichlorohydrex Glycine Powder, perfume and polyacrylate microcapsule are added to the tank. The composition is mixed here for at least 15 minutes before it is cooled to 50-55°C and poured into canisters.

Table 1

| | Example A | Example B | Example C (comparative) |
|---|---|---|---|
| Aluminum Zirconium Trichlorohydrex Glycine Powder | 24 | 24 | 24 |
| Cyclopentasiloxane | QS | QS | QS |
| Dimethicone* | - | - | - |
| CO-1897 Stearyl Alcohol NF | 14 | 14 | 14 |
| Hydrogenated Castor Oil MP80 Deodorized | 3.85 | 3.85 | 3.85 |
| Behenyl Alcohol | 0.2 | 0.2 | 0.2 |
| Tribehenin | - | - | - |
| C 18 - 36 acid triglyceride | - | - | - |
| C12-15 Alkyl Benzoate* | 9.5 | 9.5 | 5 |
| PPG-14 Butyl Ether* | 6.5 | 6.5 | - |
| Phenyl Trimethicone* | 3 | - | - |
| White Petrolatum* | 3 | - | - |
| Mineral Oil* | 1.0 | 1.0 | 1.0 |
| Fragrance | 0.75 | 0.75 | 0.75 |
| Talc Imperial 250 USP | 3.0 | 3.0 | 3.0 |
| Polyacrylate Microcapsule | 1.9 | 1.9 | 1.9 |
| QS - indicates that this material is used to bring the total to 100%.<br>* - indicates the non-volatile oils. | | | |

Following, Table 2 discloses the percentages of non-volatile oils for Examples A, B, and C from Table 1. For each of these examples, Table 2 also shows the increase in headspace value, when measured according to the Headspace Test Method and the change in color lightness when measured according to Residue Evaluation Test Method. Further, Table 2 also includes related data from the Sensory Test Method, which are intended to validate the other data; specifically, the data for Fragrance at 90 minutes are intended to validate the data for Znose % increase, and the data for White Residue at application are intended to validate the data for change in color lightness. As shown in Table 2, there are good correlations between these data sets. This data appear to indicate that for compositions with lower percentages of non-volatile oils, there are significantly greater increases in headspace value. With regard to change in color lightness, Examples A and B demonstrate acceptable levels of change, however Example C demonstrates a less desirable level of change; that is, Example C exhibits more white residue at application.

Table 2

| Example Composition | A | B | C |
|---|---|---|---|
| % Non Volatile Oils | 23 | 17 | 6 |
| Znose % Increase (increase in headspace value when measured according to the Headspace Test Method) | 3,799% | 4,367% | 6,206% |
| $\Delta L^*$ at Application (change in color lightness when measured according to Residue Evaluation Test Method) | 4.43c | 5.29b | 6.26a |
| White Residue at Application (Sensory Test Method) | 2.6 b | 2.9 b | 3.4 a |
| Fragrance at 90 minutes post rub (Sensory Test Method) | 1.7 b | 2.3 a | 2.2 a |

Following, in Table 3, are further Examples of antiperspirant compositions. Examples G and H are invisible solid anhydrous antiperspirant compositions including low percentages of non-volatile oils and microcapsules made by interfacial polymerization, wherein the microcapsules encapsulate a fragrance.

Table 3

| | **Example G** | **Example H** |
|---|---|---|
| Aluminum Zirconium Trichlorohydrex Glycine Powder | 25.6 | 25.6 |
| Cyclopentasiloxane | QS | QS. |
| CO-1897 Stearyl Alcohol NF | 13 | 13 |
| Hydrogenated Castor Oil MP80 Deodorized | 2.9 | 2.9 |
| Behenyl Alcohol | 0.2 | 0.2 |
| Ozokerite Wax SP-1026 | 1.0 | 1.0 |
| C12-15 Alkyl Benzoate* | 8.5 | 8.5 |
| PPG-14 Butyl Ether* | 6.5 | 6.5 |
| Mineral Oil* | 1.0 | 1.0 |
| Fragrance | 0.75 | 0.75 |
| Talc Imperial 250 USP | 2.5 | 2.5 |
| Polyacrylate Microcapsule | 1.5 | 2.5 |
| Fragrance Complexed Beta-cyclodextrin | 3 | 3 |
| DL-ALPHA Tocopheryl Acetate (Vitamin E) | 0.1 | 0.1 |
| d-Panthenyl Triacetate | 0.1 | 0.1 |
| Acetyl Glucosamine | 0.1 | 0.1 |
| QS - indicates that this material is used to bring the total to 100%.<br>* - indicates the non-volatile oils. | | |

## Claims

1. An anhydrous composition, comprising:

   a plurality of microcapsules, wherein the microcapsules comprise a core material and a shell encapsulating the core material;
   from 10% to 23%, by total mass of the anhydrous composition, of non-volatile oils; wherein the core material comprises a first fragrance; and wherein the shell includes a polyacrylate material;
   wherein the microcapsules are spray-dried microcapsules; and
   wherein the anhydrous composition further comprises a moisture-triggered fragrance delivery system.

2. The anhydrous composition according to claim 1, wherein the shell of the microcapsules has a fracture strength of from 0.2 mega Pascals to 10.0 mega Pascals, preferably from 0.2 mega Pascals to 2.0 mega Pascals, when measured according to the Fracture Strength Test Method as disclosed herein.

3. The anhydrous composition according to any preceding claim, wherein the polyacrylate material forms at least 33% of the overall mass of the shell.

4. The anhydrous composition according to any preceding claim, further comprising volatile oils that form 10% to 65%, preferably from 20% to 40%, of the total mass of the anhydrous composition.

5. The anhydrous composition according to any preceding claim, wherein the non-volatile oils comprise a material selected from the group consisting of emollients, residue maskers, mineral oils, and combinations thereof.

6. The anhydrous composition according to any preceding claim, wherein the fragrance is selected from animal fragrances such as musk oil, civet, castoreum, ambergris, plant fragrances such as nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof

7. The anhydrous composition according to any preceding claim, wherein the anhydrous composition is a solid antiperspirant composition which further includes a volatile fluid being a volatile cyclic silicone.

8. The anhydrous composition according to any preceding claim, wherein the non-volatile oils are selected from the group consisting of: dimethicone, C12-15 akyl benzoate, PPG-14 butyl ether, phenyl trimethicone, isopropyl myristate, 2-phenyl ethyl benzoate, and combinations thereof

9. The anhydrous composition according to any preceding claim, further comprising a parent fragrance dispersed throughout the anhydrous composition; wherein the first fragrance is a non-parent fragrance.

10. The anhydrous composition according to any preceding claim, wherein the moisture-triggered fragrance delivery system is a spray-dried cyclic oligosaccharide.

11. The anhydrous composition according to any preceding claim, wherein the anhydrous composition further comprises:

    from 0.1% to 30% by weight of the anhydrous composition, of one or more antiperspirant actives; a
    from 0.1% to 35% by weight of the anhydrous composition, of one or more structurants; and
    from 10% to 99% by weight of the anhydrous composition, of one or more anhydrous liquid carriers.

**Patentansprüche**

1. Wasserfreie Zusammensetzung, die Folgendes umfasst:

    eine Vielzahl von Mikrokapseln, wobei die Mikrokapseln ein Kernmaterial und eine das Kernmaterial einkapselnde Hülle umfasst;
    von 10 % bis 23 %, nach Gesamtmasse der wasserfreien Zusammensetzung, nichtflüchtige Öle;
    wobei das Kernmaterial einen ersten Duftstoff umfasst; und wobei die Hülle ein Polyacrylatmaterial einschließt;
    wobei die Mikrokapseln sprühgetrocknete Mikrokapseln sind; und
    wobei die wasserfreie Zusammensetzung ferner ein durch Feuchtigkeit ausgelöstes Duftstoff-Abgabesystem umfasst.

2. Wasserfreie Zusammensetzung nach Anspruch 1, wobei die Hülle der Mikrokapseln eine Bruchfestigkeit von 0,2 Mega-Pascal bis 10,0 Mega-Pascal, vorzugsweise von 0,2 Mega-Pascal bis 2,0 Mega-Pascal bei Messung nach dem hier offenbarten Bruchfestigkeits-Prüfverfahren aufweist.

3. Wasserfreie Zusammensetzung nach einem der vorstehenden Ansprüche, wobei das Polyacrylatmaterial mindestens 33 % der Gesamtmasse der Hülle ausmacht.

4. Wasserfreie Zusammensetzung nach einem der vorstehenden Ansprüche, die ferner flüchtige Öle umfasst, die 10 % bis 65 %, vorzugsweise von 20 % bis 40 %, der Gesamtmasse der wasserfreien Zusammensetzung ausmachen.

5. Wasserfreie Zusammensetzung nach einem der vorstehenden Ansprüche, wobei die nichtflüchtigen Öle ein Material ausgewählt aus Weichmachern, Rückstands-Maskiermitteln, Mineralölen und Kombinationen davon umfassen.

6. Wasserfreie Zusammensetzung nach einem der vorstehenden Ansprüche, wobei der Duftstoff ausgewählt ist aus tierischen Duftstoffen wie Moschusöl, Zibet, Bibergeil, Amber, Pflanzenduftstoffe wie Muskatextrakt, Kardamomextrakt, Ingwerextrakt, Zimtextrakt, Patschuliöl, Geranienöl, Orangenöl, Mandarinenöl, Orangenblumenöl, Zedernholz, Vetiver, Lavandin, Ylangextrakt, Tuberoseextrakt, Sandelholzöl, Bergamottöl, Rosmarinöl, Grüne-Minze-Öl, Pfefferminzöl, Zitronenöl, Lavendelöl, Zitronellöl, Kamillenöl, Gewürznelkenöl, Salbeiöl, Neroliöl, Labdanumöl, Eukalyptusöl, Eisenkrautöl, Mimosenextrakt, Narzissenextrakt, Karottensamenextrakt, Jasminextrakt, Olibanumextrakt, Rosenextrakt und Mischungen davon

7. Wasserfreie Zusammensetzung nach einem der vorstehenden Ansprüche, wobei die wasserfreie Zusammensetzung eine feste Antitranspirant-Zusammensetzung ist, die ferner ein flüchtiges Fluid umfasst, das ein flüchtiges zyklisches Silikon ist.

8. Wasserfreie Zusammensetzung nach einem der vorstehenden Ansprüche, wobei die nichtflüchtigen Öle ausgewählt sind aus der Gruppe bestehend aus: Dimethicon, C12-15-Akylbenzoat, PPG-14-Butylether, Phenyltrimethicon, Isopropylmyristat, 2-Phenylethylbenzoat und Kombinationen davon

9. Wasserfreie Zusammensetzung nach einem der vorstehenden Ansprüche, die ferner einen Stamm-Duftstoff umfasst, der innerhalb der wasserfreien Zusammensetzung verteilt ist; wobei der erste Duftstoff ein Nicht-Stamm-Duftstoff ist.

10. Wasserfreie Zusammensetzung nach einem der vorstehenden Ansprüche, wobei das durch Feuchtigkeit ausgelöste Duftstoff-Abgabesystem ein sprühgetrocknetes zyklisches Oligosaccharid ist.

11. Wasserfreie Zusammensetzung nach einem der vorstehenden Ansprüche, wobei die wasserfreie Zusammensetzung ferner Folgendes umfasst:

von 0,1 Gew.-% bis 30 Gew.-% der wasserfreien Zusammensetzung, eines oder mehrerer Antitransparent-Wirkstoffe; a
von 0,1 Gew.-% bis 35 Gew.-% der wasserfreien Zusammensetzung, einer oder mehreren Strukturen; und
von 10 Gew.-% bis 99 Gew.-% der wasserfreien Zusammensetzung, einer oder mehrerer wasserfreier flüssiger Träger.

**Revendications**

1. Composition anhydre, comprenant :

une pluralité de microgélules, dans laquelle les microgélules comprennent un matériau de noyau et une enveloppe encapsulant le matériau de noyau ;
de 10 % à 23 %, en masse totale de la composition anhydre, d'huiles non volatiles ;
dans laquelle le matériau de noyau comprend un premier parfum ; et dans laquelle l'enveloppe inclut un matériau de polyacrylate ;
dans laquelle les microgélules sont des microgélules séchées par atomisation ; et
où la composition anhydre comprend en outre un système de libération de parfum à déclenchement par l'humidité.

2. Composition anhydre selon la revendication 1, dans laquelle l'enveloppe des microgélules a une résistance à la rupture allant de 0,2 mégapascal à 10,0 mégapascals, de préférence de 0,2 mégapascal à 2,0 mégapascals, lorsqu'on mesure selon le procédé de test de résistance à la rupture tel que décrit ici.

3. Composition anhydre selon l'une quelconque revendication précédente, dans laquelle le matériau de polyacrylate forme au moins 33 % de la masse globale de l'enveloppe.

4. Composition anhydre selon l'une quelconque revendication précédente, comprenant en outre des huiles volatiles qui forment 10 % à 65 %, de préférence de 20 % à 40 %, de la masse totale de la composition anhydre.

5. Composition anhydre selon l'une quelconque revendication précédente, dans laquelle les huiles non volatiles comprennent un matériau choisi dans le groupe constitué d'émollients, masqueurs de résidus, huiles minérales, et leurs combinaisons.

6. Composition anhydre selon l'une quelconque revendication précédente, dans laquelle le parfum est choisi parmi des parfums animaux tels que l'huile de musc, la civette, le castoréum, l'ambre gris, des parfums de plante tels que l'extrait de noix de muscade, l'extrait de cardamome, l'extrait de gingembre, l'extrait de cannelle, l'huile de patchouli,

l'huile de géranium, l'huile d'orange, l'huile de mandarine, l'extrait de fleur d'oranger, le bois de cèdre, le vétiver, la lavande, l'extrait d'ylang, l'extrait de tubéreuse, l'huile de bois de santal, l'essence de bergamote, l'essence de romarin, l'essence de menthe verte, l'essence de menthe poivrée, l'essence de citron, l'essence de lavande, l'huile de citronnelle, l'huile de camomille, l'huile de trèfle, l'huile de sauge, l'huile de camphre, l'huile de laudanum, l'huile d'eucalyptus, l'huile de verveine, l'extrait de mimosa, l'extrait de narcisse, l'extrait de graine de carotte, l'extrait de jasmin, l'extrait d'oliban, l'extrait de rose et leurs mélanges.

**7.** Composition anhydre selon l'une quelconque revendication précédente, où la composition anhydre est une composition anti-transpiration solide qui inclut en outre un fluide volatil qui est une silicone cyclique volatile.

**8.** Composition anhydre selon l'une quelconque revendication précédente, dans laquelle les huiles non volatiles sont choisies dans le groupe constitué de : diméthicone, benzoate d'alkyle en C12 à 15, éther butylique de PPG-14, phényl-triméthicone, myristate d'isopropyle, benzoate de 2-phényléthyle, et leurs combinaisons

**9.** Composition anhydre selon l'une quelconque revendication précédente, comprenant en outre un parfum parent dispersé sur l'ensemble de la composition anhydre ; dans laquelle le premier parfum est un parfum non parent.

**10.** Composition anhydre selon l'une quelconque revendication précédente, dans laquelle le système de libération de parfum à déclenchement par l'humidité est un oligosaccharide cyclique séché par atomisation.

**11.** Composition anhydre selon l'une quelconque revendication précédente, où la composition anhydre comprend en outre :

de 0,1 % à 30 % en poids de la composition anhydre, d'un ou plusieurs agents actifs anti-transpiration ; a
de 0,1 % à 35 % en poids de la composition anhydre, d'un ou plusieurs structurants ; et
de 10 % à 99 % en poids de la composition anhydre, d'un ou plusieurs véhicules liquides anhydres ;

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2010104612 A1
- GB 2274989 A
- JP 2009280533 A
- DE 102008035013 A1
- DE 102008035014 A1
- EP 2433617 A2
- US 5578563 A
- US 4145184 A
- US 4209417 A
- US 4515705 A
- US 4152272 A
- US 6835373 B, Kolodzik
- PT 514189


### Non-patent literature cited in the description


- **ZHANG, Z. et al.** Mechanical strength of single microcapsules determined by a novel micromanipulation technique. *J. Microencapsulation,* 1999, vol. 16 (1), 117-124
- **SUN, G. ; ZHANG, Z.** Mechanical Properties of Melamine-Formaldehyde microcapsules. *J. Microencapsulation,* 2001, vol. 18 (5), 593-602
- *CIE,* 1976